# EUROPEAN PATENT APPLICATION

(11) **EP 4 542 556 A1**
(43) Date of publication of application: **23.04.2025**
(21) Application number: 23386103.8
(22) Date of filing: 20.10.2023
(51) Int. Cl.: G16H 20/00

(54) **METHOD FOR CALCULATING THE DAY OF INDUCTION OF FINAL OOCYTE MATURATION USING VOLUMETRIC CRITERIA, AIMING TO MAX THE NUMBER OF RETRIEVED MATURE OOCYTES IN IVF**

(71) Applicant: Lainas, Trifon, 11 528 Athens (GR)
(72) Inventor: Lainas, Trifon, 11 528 Athens (GR)

(57) **Abstract**

The proposed method consists of measuring as accurately as possible the volume of the follicles (but also their average diameter), through a three-dimensional (3D) ultrasound assessment. These measurements, combined with same-day hormonal measurements, help determine the optimal day of induction of final oocyte maturation.

Given that the shape of the follicles is not exclusively spherical but varies, the estimation of the volume of the follicles, through three-dimensional (3D) ultrasound evaluation, provides more reliable measurements, with the obvious result of drawing safer conclusions to determine the day of induction of final oocyte maturation.

The new method is based on the use of follicular volume criteria as determined by three-dimensional ultrasound assessment, to select the optimal day for induction, instead of the internationally established two-dimensional (x, y) traditional dimensional criteria.

## Description

In patients undergoing in vitro fertilization (IVF), the cumulative birth rate (CLBR) is closely related to the number of oocytes retrieved from the multiple follicles developed (Devesa et al., 2018; Drakopoulos et al., 2016; Polyzos et al., 2018).

The recovery of oocytes from multiple follicles is done by the method of transvaginal ultrasound-guided ovulation and follows the controlled stimulation of the woman's ovaries (Controlled Ovarian Stimulation - COS). COS, which aims at the development of multiple follicles, is achieved through the use of pharmaceutical protocols that, on the one hand, cause multiple follicle development, and on the other prevent premature and untimely rupture of the follicles (follicular ovulation), with consequent loss of oocytes (Olivennes et al., 2000). The most commonly used is the flexible competitor protocol (Lainas et al., 2005).

The monitoring of the multiple follicle development is done with a series of ultrasounds and hormonal tests, which determine the day of reevaluation, the continuation and/or modification of the treatment regimen. The success of this phase of the program depends on frequent and accurate ultrasound monitoring of the ovarian response to the treatment regimen. When monitoring controlled ovarian stimulation, transvaginal ultrasound is an important aid in monitoring the course of stimulation. Based on ultrasound and hormonal criteria, the optimal day for triggering of final oocyte maturation is selected.

To date, the accepted ultrasound criteria for induction of final oocyte maturation is the presence of three follicles ≥17mm in diameter (Kolibianakis et al., 2004). Traditionally, follicular development is monitored by a two-dimensional ({x,y} - 2D) ultrasound assessment. The number and dimensions (x,y) of developing follicles in each ovary are measured. The above method obviously depends on the skill and experience of the operator, while large discrepancies have been observed in the measurements performed by different operators (inter-observer variability) but also by the same operator (intra-observer variability). The technical difficulty of accurate ultrasound monitoring/estimation increases rapidly as the number of developing follicles increases, resulting in the possible incorrect selection of the day of induction of final oocyte maturation.

The new generation ultrasounds also offer three-dimensional (3D) imaging using systems for automatic identification and measurement of follicle dimensions. Software is used that analyzes the data retrieved through the three-dimensional (3D) ultrasound assessment, identifying and quantifying the size of any subsonic area individually. Thus, an automatic estimation of their dimensions and volume is provided.

The herein proposed method concerns measuring, with the maximum possible accuracy, the volume of the follicles (but also their average diameter {x,y,z}), through three-dimensional (3D) ultrasound assessment. These measurements, combined with hormonal measurements of the same day, are used as a method of determining the optimal day to induce the final oocyte maturation, with the aim of obtaining the maximum possible number of mature oocytes.

Given that the shape of the follicles is not exclusively spherical, but varies significantly, the use of volumetric criteria (ml) may lead to safer conclusions for the determination of the induction day of the final oocyte maturation, in contrast to the internationally established two-dimensional 2D dimensional {x, y} criteria, which do not take into account the third (z) dimension. Measurements from three-dimensional (3D) ultrasound assessment can be upgraded in accuracy in determining the optimal day to induce final oocyte maturation through an algorithm that also combines demographic, embryological, clinical, and ovarian stimulation data with or without assistance from a convolutional neural network. Specifically, the number of follicles with a volume ≥0.5 ml and an average diameter >11mm as well as the number of those with a volume ≥2.5ml and an average diameter >17mm are included in the algorithm. The above parameters, as well as the levels of estradiol, progesterone, luteinizing hormone (LH) and follicle stimulating hormone (FSH) can compose a predictive model that will provide a more accurate recommendation for induction of final oocyte maturation.

### Bibliography

Devesa, M., Tur, R., Rodriguez, I., Coroleu, B., Martinez, F., & Polyzos, N. P. (2018). Cumulative live birth rates and number of oocytes retrieved in women of advanced age. A single centre analysis including 4500 women ≥38 years old. Human Reproduction, 33(11), 2010-2017. https://doi.org/10.1093/humrep/dey295

Drakopoulos, P., Blockeel, C., Stoop, D., Camus, M., de Vos, M., Tournaye, H., & Polyzos, N. P. (2016). Conventional ovarian stimulation and single embryo transfer for IVF/ICSI. How many oocytes do we need to maximize cumulative live birth rates after utilization of all fresh and frozen embryos? Human Reproduction, dev316. https://doi.org/10.1093/humrep/dev316

Kolibianakis, E. M., Albano, C., Camus, M., Tournaye, H., Van Steirteghem, A. C., & Devroey, P. (2004). Prolongation of the follicular phase in in vitro fertilization results in a lower ongoing pregnancy rate in cycles stimulated with recombinant follicle-stimulating hormone and gonadotropin-releasing hormone antagonists. Fertility and Sterility, 82(1), 102-107. https://doi.org/10.1016/i.fertnstert.2004.01.027

Lainas, T., Zorzovilis, J., Petsas, G., Stavropoulou, G., Cazlaris, H., Daskalaki, V., Lainas, G., & Alexopoulou, E. (2005). In a flexible antagonist protocol, earlier, criteria-based initiation of GnRH antagonist is associated with increased pregnancy rates in IVF. Human Reproduction, 20(9), 2426-2433. https://doi.org/10.1093/humrep/dei106

Olivennes, F., Belaisch-Allart, J., Emperaire, J.-C., Dechaud, H., Alvarez, S., Moreau, L., Nicollet, B., Zorn, J.-R., Bouchard, P., & Frydman, R. (2000). Prospective, randomized, controlled study of in vitro fertilization-embryo transfer with a single dose of a luteinizing hormone-releasing hormone (LH-RH) antagonist (cetrorelix) or a depot formula of an LH-RH agonist (triptorelin). Fertility and Sterility, 73(2), 314-320. https://doi.org/10.1016/S0015-0282(99)00524-5 Polyzos, N. P., Drakopoulos, P., Parra, J., Pellicer, A., Santos-Ribeiro, S., Tournaye, H., Bosch, E., & Garcia-Velasco, J. (2018). Cumulative live birth rates according to the number of oocytes retrieved after the first ovarian stimulation for in vitro fertilization/intracytoplasmic sperm injection: A multicenter multinational analysis including ~15,000 women. Fertility and Sterility, 110(4), 661-670.e1. https://doi.org/10.1016/i.fertnstert.2018.04.039

## Claims

1. Method for calculating the day of induction of final oocyte maturation using volumetric criteria, which will replace the traditional two-dimensional dimensional criteria, aiming to maximize the number of obtained mature oocytes in the context of in vitro fertilization, **characterized by** the existence of at least 3 follicles with an average volume greater than 2.5ml, as calculated based on 3D ultrasound assessment and the use of machine learning technologies to accurately determine the day of induction of oocyte maturation.

2. Method for calculating the day of induction of final oocyte maturation of claim 1, **characterized by** establishing three-dimensional mean follicular diameter criteria {x, y, z} >11mm and >17mm.

3. Method for calculating the day of induction of the final oocyte maturation of claims 1-2, **characterized by** the combination of hormonal criteria (estradiol, progesterone and luteinizing hormone {LH}), volumetric criteria and dimensional criteria with the aim of maximizing the number of mature oocytes obtained.
